# EUROPEAN PATENT SPECIFICATION

(11) **EP 2 886 539 B1**
(45) Date of publication and mention of the grant of the patent: **01.02.2017**
(21) Application number: 14460003.8
(22) Date of filing: 06.02.2014
(51) Int. Cl.: C07D 317/70, C07D 409/04

(54) **10-Thiosubstituted pentahydroxyanthracene derivatives, a method of their preparation and intermediate compounds**
10-thiosubstituierte Pentahydroxyanthracenderivate, Verfahren zu deren Herstellung und Zwischenverbindungen
Dérivés de pentahydroxyanthracene 10-thiosubstitués, leur procédé de préparation et composés intermédiaires

(30) Priority: 19.12.2013 PL 40660013
(43) Date of publication of application: 24.06.2015
(73) Proprietor: Centrum Badan Molekularnych I Makromolekularnych Polskiej Akademii Nauk, 90-363 Lodz (PL)
(72) Inventor: Balczewski, Piotr, 91-225 Lodz (PL); Kowalska, Emilia, 96-100 Skierniewice (PL); Skalik, Joanna, 42-270 Klomnice (PL)
(74) Representative: Brodowska, Iwona

(56) References cited:
- PIOTR BALCZEWSKI ET AL: "Unusual Transformation of the Diarylmethanol Derivative into an Unknown 1,2,3,6,7,10-Hexahydroxylated Anthracene System", THE JOURNAL OF ORGANIC CHEMISTRY, vol. 71, no. 7, 1 March 2006 (2006-03-01), pages 2899-2902, XP055079452, ISSN: 0022-3263, DOI: 10.1021/jo052599x
- AGNIESZKA BODZIOCH ET AL: "Synthesis and Optoelectronic Properties of Hexahydroxylated 10-O-RSubstituted Anthracenes via a New Modification of the Friedel-Crafts Reaction Using O-Protected ortho-Acetal Diarylmethanols", CHEMISTRY- A EUROPEAN JOURNAL, vol. 18, 21 March 2012 (2012-03-21), pages 4866-4876, XP55049553, DOI: 10.1002/chem.201101909

## Description

### Technical Field

The invention relates to new 10-thiosubstituted pentahydroxyanthracene derivatives represented by the general formula 1, 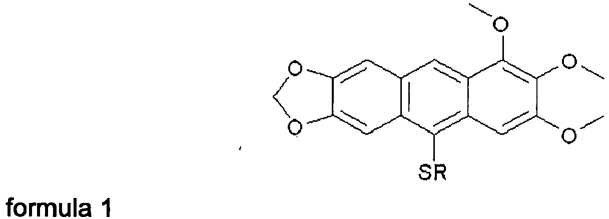 wherein R- is - alkyl having n=1 to 12 carbon atoms, especially benzyl, phenyl or naphthyl, and their method of preparation.

The invention also relates to intermediates of the formula 2, which similarly to the compounds of the formula 1 may be used in organic optoelectronics.

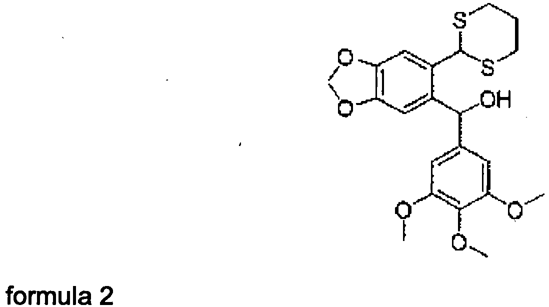

The invention also relates to the process for preparation of fused hydrocarbons represented by the formula 1, based on the reaction of transformation of derivatives of the formula 2 in the presence of FeCl₃ and Kl, which is carried out in boiling methanol, in a single reaction vessel, without isolation of intermediates.

### State of the art

Anthracene and its substituted derivatives, due to its photoluminescent and electroluminescent properties, are of great importance in optoelectronics. They are chemically durable materials which emit a blue light. They are used in optoelectronic devices, such as OLED, OPV, OFET.

Recent literature reports indicate that the introduction of a organosulfur substituent to the anthracene molecule in place of the analogous oxygen substituent (OR exchange to SR) significantly influences the change of interaction and orientation of molecules in the solid, which is presumed, it must also lead to changes in the optoelectrical properties¹.

The literature describes various reactions leading to receive thiosubstituted anthracenes starting from substrates containing three fused rings:
1) sulfenylation reaction of anthracenes², alkylation of anthracenethiols³, oxidation of anthracenethiols to bisanthryldisulfides⁴;
2) the exchange reaction of functional groups such as OH⁵, OMe⁶, OAc⁷, Br⁸, Cl⁹, NO₂¹⁰, I¹¹ in anthracenes into organosulfur groups;
3) the conversion of the anthraquinones¹² or tioanthraquinones¹³ into anthracenes substituted with a group SR at the position 10;

There are not known methods of synthesis of thiosubstituted anthracenes, of the invention, from substrates not containing three fused rings.

The organosulfur compounds of formulae **1a-d**, as well as intermediate compounds of formulae **2a-d** may be used in optoelectronic devices as the new generation of organic, multilayer light-emitting diodes (OLED)¹⁴, organic highfield transistors (OFET)¹⁵, and other devices using organic semi- and photoconductors¹⁶ based on the use of other organosulfur derivatives. Furthermore, the above mentioned compounds of the formula 1 may provide a basis for further modification and synthesis of other optoelectronic materials.

The compounds of the general the formula 1 may also have biological activity. In the literature, the thioanthracenes derivatives have found use as precursors of pesticides and pharmaceuticals intermediates, such as antioxidants¹⁷ .

### Summary of invention

The invention relates to new 10-thiosubstituted pentahydroxyanthracene derivatives represented by the general formula **1,** 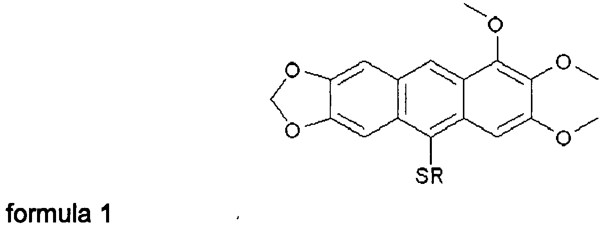 wherein R- is - alkyl having n=1 to 12 carbon atoms, especially benzyl, phenyl or naphthyl, and their method of preparation.

The compounds of the formula 1 preferably are performed by formulas **1a-1 d.**

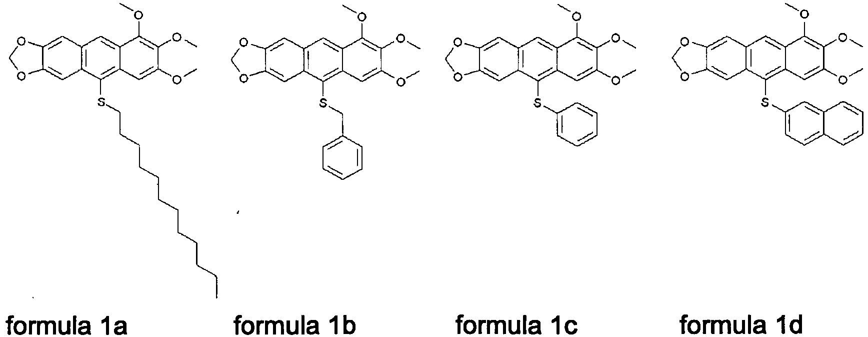

Intermediates of the formula **2,** wherein R- is - alkyl having n=1 to 12 carbon atoms, especially benzyl, phenyl or naphthyl.

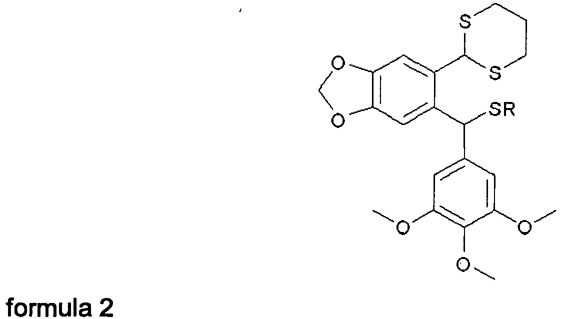

Intermediates of the formula 2 preferably are presented in formulae 2a - 2d.

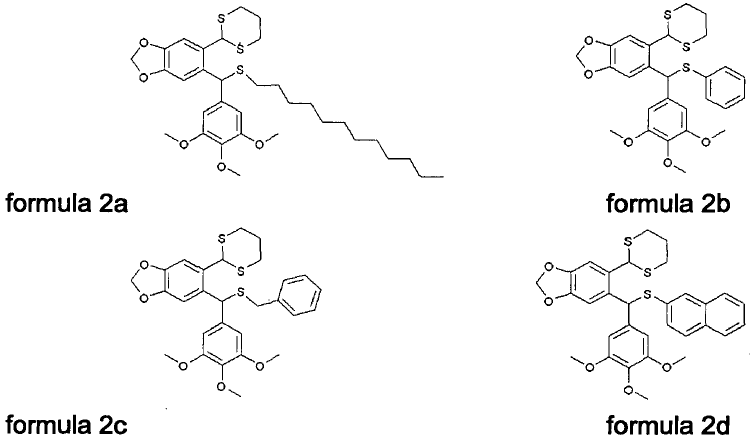

A method for producing fused aromatic hydrocarbons of the formula **1,** wherein R is as defined above, according to the invention is that is carried out the reaction of transformation of diaryl derivatives of the formula **2,**

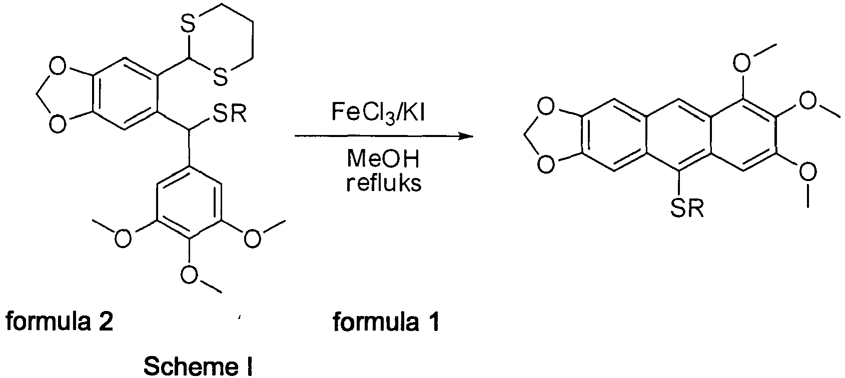

in the presence of iron trichloride, and potassium iodide in the environment of boiling organic solvent, especially an aliphatic alcohol, in a single reaction vessel, without isolation of intermediate products, to the derivatives of the formula **1** containing the new six-membered aromatic ring with a group SR at position 10. The reaction is generally illustrated in Scheme I.

In the present invention, preferably is carried out the transformation of diaryl derivatives of the formula **2a-d,** in which the substituents are as defined above, in an boiling organic solvent, in a single reaction vessel, without isolation of intermediate products, to the derivatives of the formula **1a-d.**

In the present invention, preferably diaryl derivative of the formula 2a-d with CHO group protected by the 1,3-dithiane block, is reacted with FeCl₃/Kl, in boiling methanol, to afford the anthracene derivatives **1a-d** in good or quantitative yields in a single reaction step.

In the present invention, the use of a mixture of FeCl₃/KI in a 1:1 ratio allows to perform the above chemical transformation (Scheme I) in a short time, efficiently and without the need to purify the final products.

In the present invention, a necessary condition for carrying out the cyclization leading to obtain anthracenes of the formula **1** is the combined use of the two reactants FeCl₃ and KI, because the reaction does not proceeds in the presence of only one of them.

A method for producing fused aromatic hydrocarbons of the formula **1,**

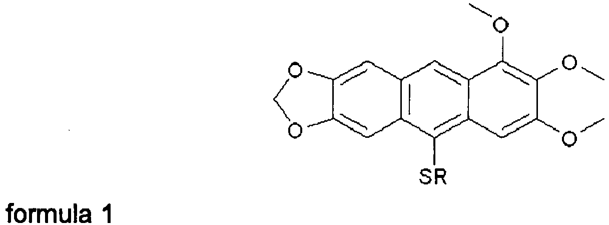

wherein R is as defined above, through the intermediates of the formula **2,** according to the invention, consists in the fact that is carried out the reaction of transformation of an aromatic aldehyde **5** containing halogen atoms in the ortho position, preferably bromine to the compound of the formula **4** with a protected carbonyl function in the form of dithioacetal, which is then transformed into a compound of the formula **3.** The compound 3 is converted to the compound of the formula **2.**

The course of the synthesis is shown in Scheme II

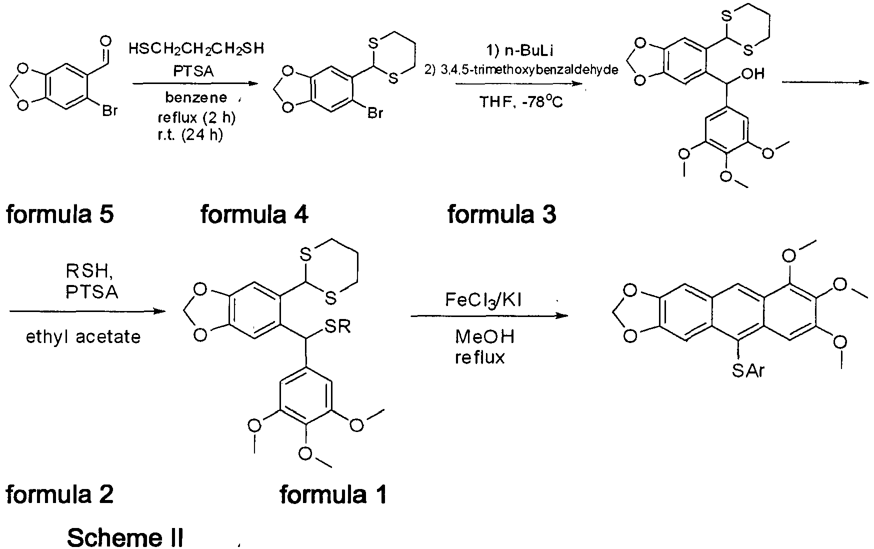

A method for preparation of aromatic hydrocarbons of the formula **1** according to the invention has the following advantages:
(a) A method for preparation of fused aromatic hydrocarbons of the formula **1** is simple to implement, without using strong Brönsted acids, one-step process.
(b) According to the invention new compounds 1 are obtained, which contains a central six-membered aromatic ring with a group SR.
(c) According to the invention, compounds of the formula **1** are obtained, in good and quantitative yields, in a fast way, using commonly available reagents, such as FeCl₃/KI in a 1:1 ratio, in an organic solvent, without further purification of the final products.
(d) The derivatives of the formula **2** may be readily prepared from the appropriate diarylmethanols derivatives of the formula **3.**

Synthesis of intermediate compounds of the formula **2** involves the use of the following reaction sequence:
1) protection of the carbonyl function by dithioacetal block in the aromatic aldehyde of the formula **5** containing in the ortho position a halogen atom, preferably bromine, in the presence of a Brönsted, a Lewis acid or ion exchange resin, preferably p-toluenesulfonic acid (PTSA).
2) replacement of the halogen atom in the ortho position to the aldehyde group in the obtained compound of the formula **4,** preferably bromine to metal, preferably Li, by a base, preferably n-butyllithium, followed by condensation with another aromatic aldehyde, preferably 3,4,5-trimethoxybenzaldehyde and acidification to obtain an alcohol of the formula **3**
3) replacement of -OH functional group in alcohol of the formula 3 to a group -SR in the compound of the formula **2,** in the presence of an acid, preferably PTSA.

As the α-bromo aromatic aldehyde, preferably 6-bromopiperonal is used and as the aromatic aldehyde, preferably 3,4,5-trimethoxybenzaldehyde is used.

As thiols (RSH) are preferably used: dodecanothiol, benzenethiol, benzylthiol and 2-naphthalenethiol.

### Experimental Procedures:

### EXAMPLE I

### Synthesis of 1-[1-(2-bromo-4,5-methylenedioxyphenyl)]-1,3-dithiane represented by the formula 4

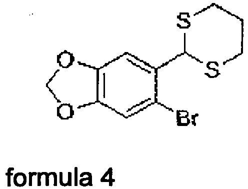

To a solution of 6-bromopiperonal of the formula **5** (Scheme II) (2.7 g, 0.012 mol) in benzene (100 mL), was added p-toluenesulfonic acid (0.8 mmol, 134 mg) and 1,3- propanedithiol (0.013 mol, 1.3 mL). The mixture was heated to reflux for 2 hours, then stirred at room temperature for 2 days. The mixture was then diluted with diethyl ether (70 mL), washed with aqueous solution of 2 M NaOH (15 mL) and H₂O (3 x 15 mL) and dried over anhydrous MgSO₄. After removal of the solvent, the product was crystallized from benzene/hexane mixture (1:1) to give 3.815 g of the product of the formula **4** (Scheme II) in 82% yield.

### EXAMPLE II

### Synthesis of (6-[1,3]-dithian-2-yl-benzo[1,3]dioxol-5-yl)-(3,4,5-trimethoxyphenyl)-methanol represented by the formula 3

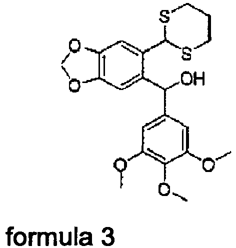

To a solution of a compound of the formula **4** (400 mg, 1.2 mmol) in dry THF (15 mL), cooled to -78°C, n-BuLi was added (1.4 eq., 2.7 M solution in hexane). The mixture was stirred at this temperature under argon for 25 minutes, then a solution of 3,4,5-trimethoxybenzaldehyde was added (261 mg, 1.3 mmol) in dry THF (8 mL). The mixture was stirred at -78°C for 2 hours, then brought to room temperature and diluted with ethyl acetate (20 mL). Next a saturated aqueous solution of NH₄Cl was added (10 mL) and the mixture was washed with H₂O (10 mL) and dried over anhydrous MgSO₄. After removal of the solvent 0.356 mg of the product of the formula **3** was obtained in 66% yield.

### EXAMPLE III

General procedure for exchange of the hydroxyl group in diarylmethanol of the formula **3** to SR group to give diarylmethanethioether of the formula **2**

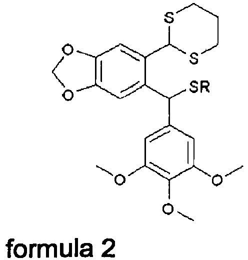

To a suspension of diarylmethanol of the formula **3** (0.3 mmol) in ethyl acetate, were added the appropriate thiol (10 eq.: dodecanothiol, benzylthiol, benzenethiol or 2-naphthylthiol) and p-toluenesulfonic acid (1.1 eq.) The mixture was stirred for 2 hours and then the mixture was diluted with ethyl acetate (15 mL), washed with 3% aqueous solution of NaOH (3 x 5 mL), H₂O (3 x 5 mL) and dried over anhydrous MgSO₄. The products were purified by column chromatography using petroleum ether: ethyl acetate (2:1) as eluent. The products with formulae 2a, 2b, 2c, 2d were obtained in the following yields: 78%, 78%, 89%, 79%.

### EXAMPLE IV

General procedure for synthesis of the RS - substituted anthracenes at the position 10 of the formula **1.**

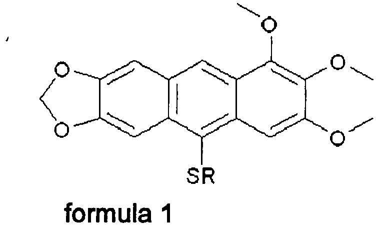

To a solution of the appropriate diarylmethanethioether of the general formula **2** (0.2 mmol) in methanol (5 mL), FeCl₃ (0.3 mmol) and KI (0.3 mmol) were added. The mixture was stirred to reflux for 2.5 hours and then for 1.5 hours at room temperature. The precipitate was washed several times with methanol (3 x 5 mL) to give the appropriate RS substituted anthracenes at the position 10 of formulae **1a, 1b, 1c, 1d,** in 53%, 84%, 82%, 85% yields, respectively.

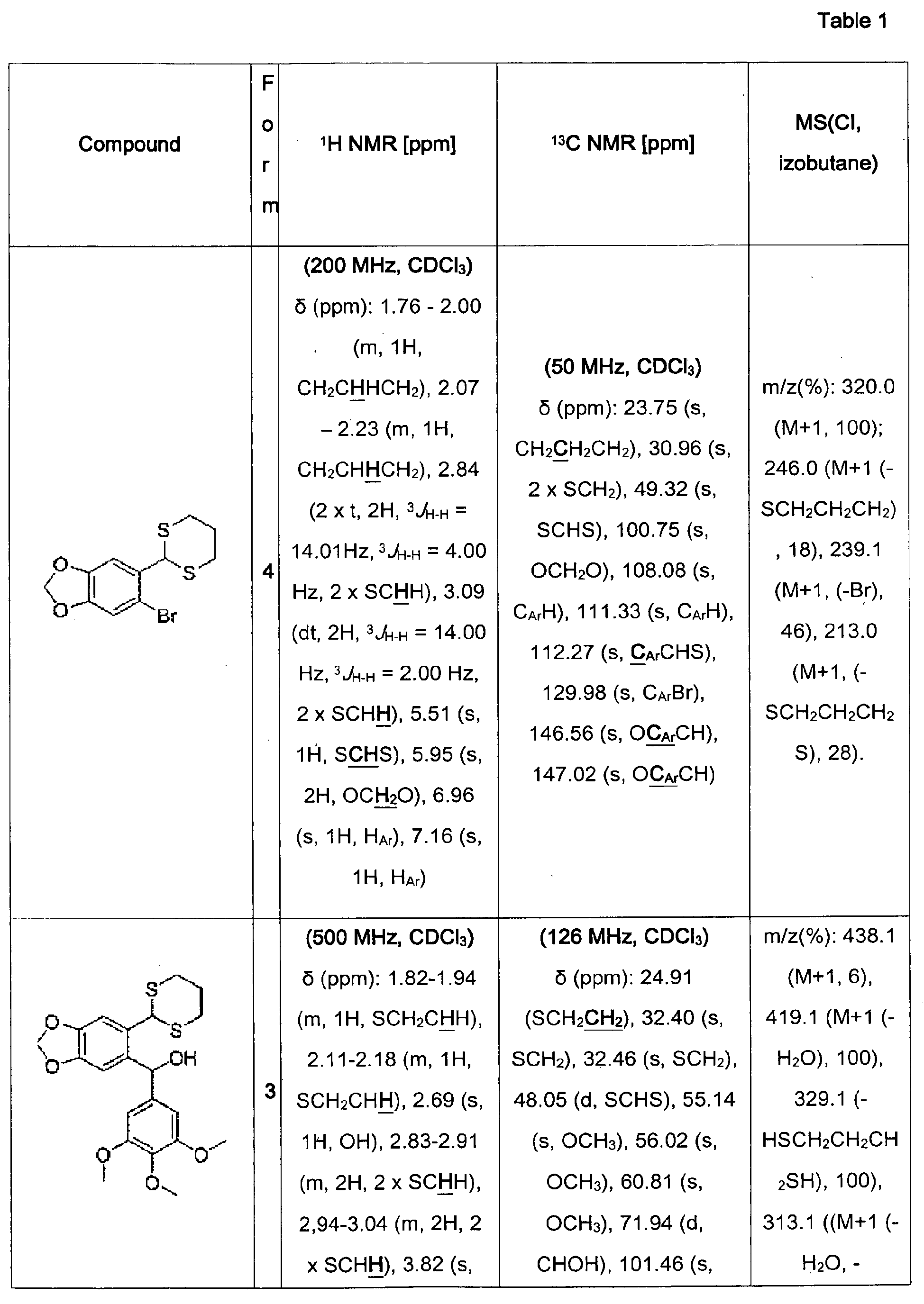

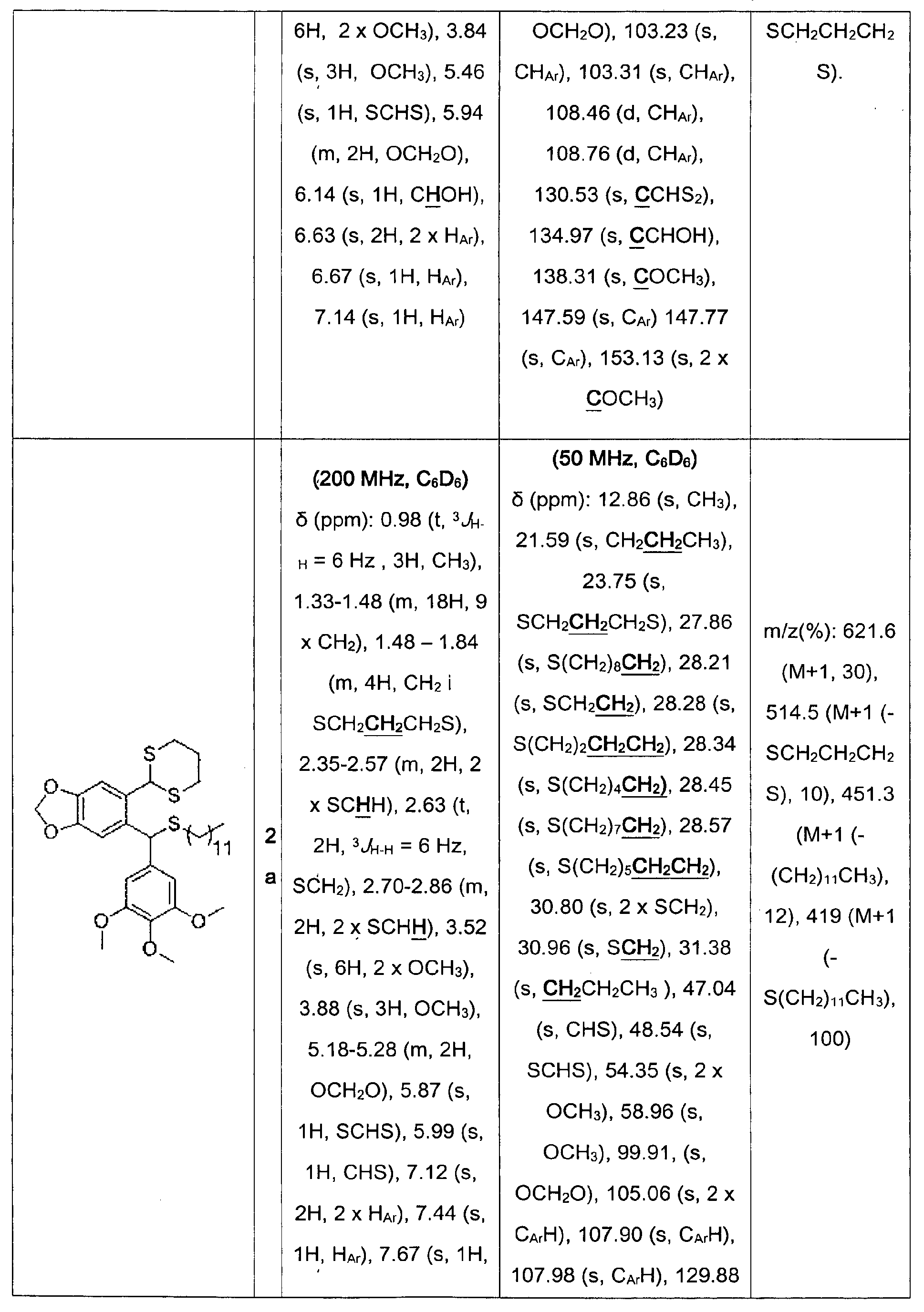

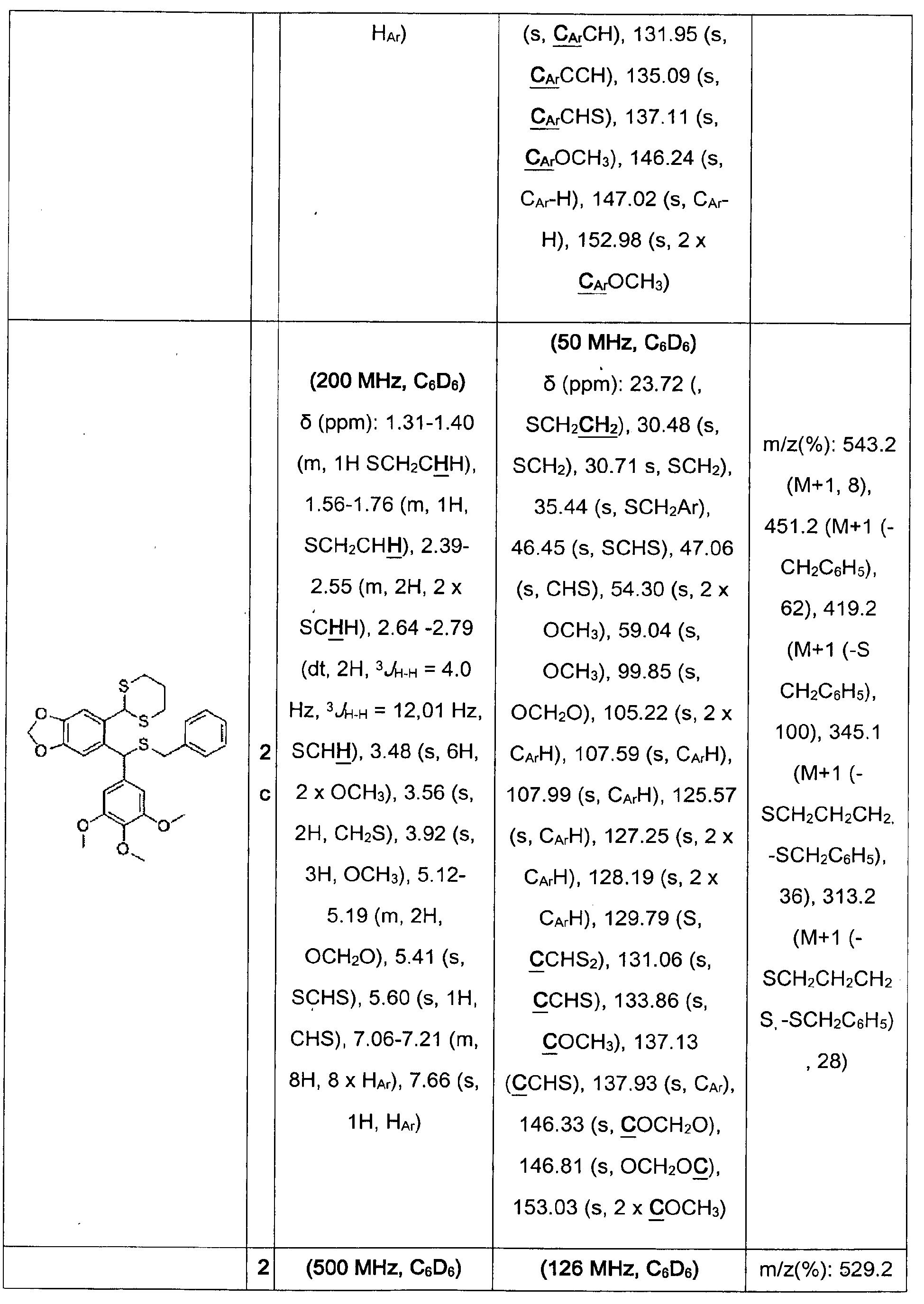

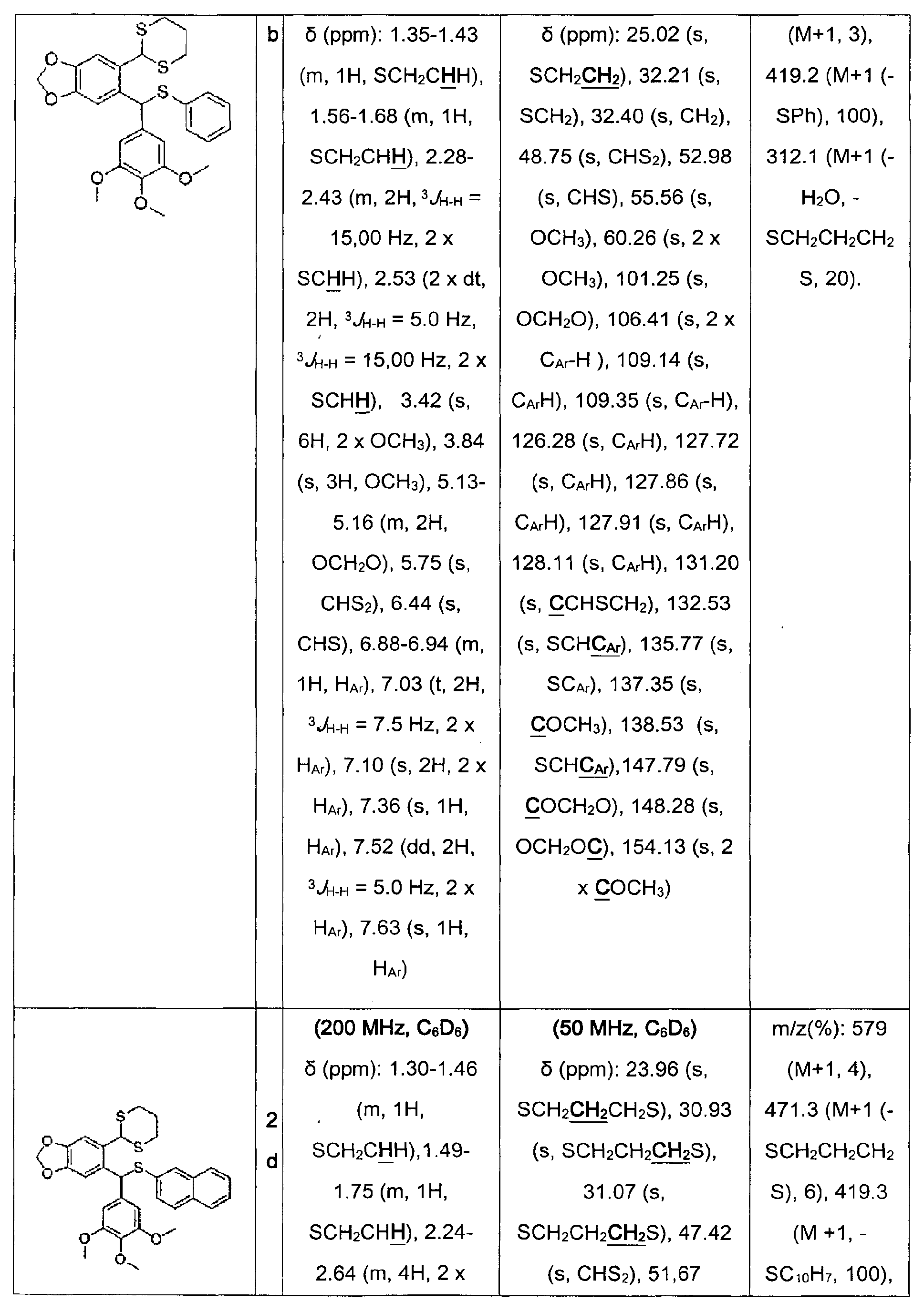

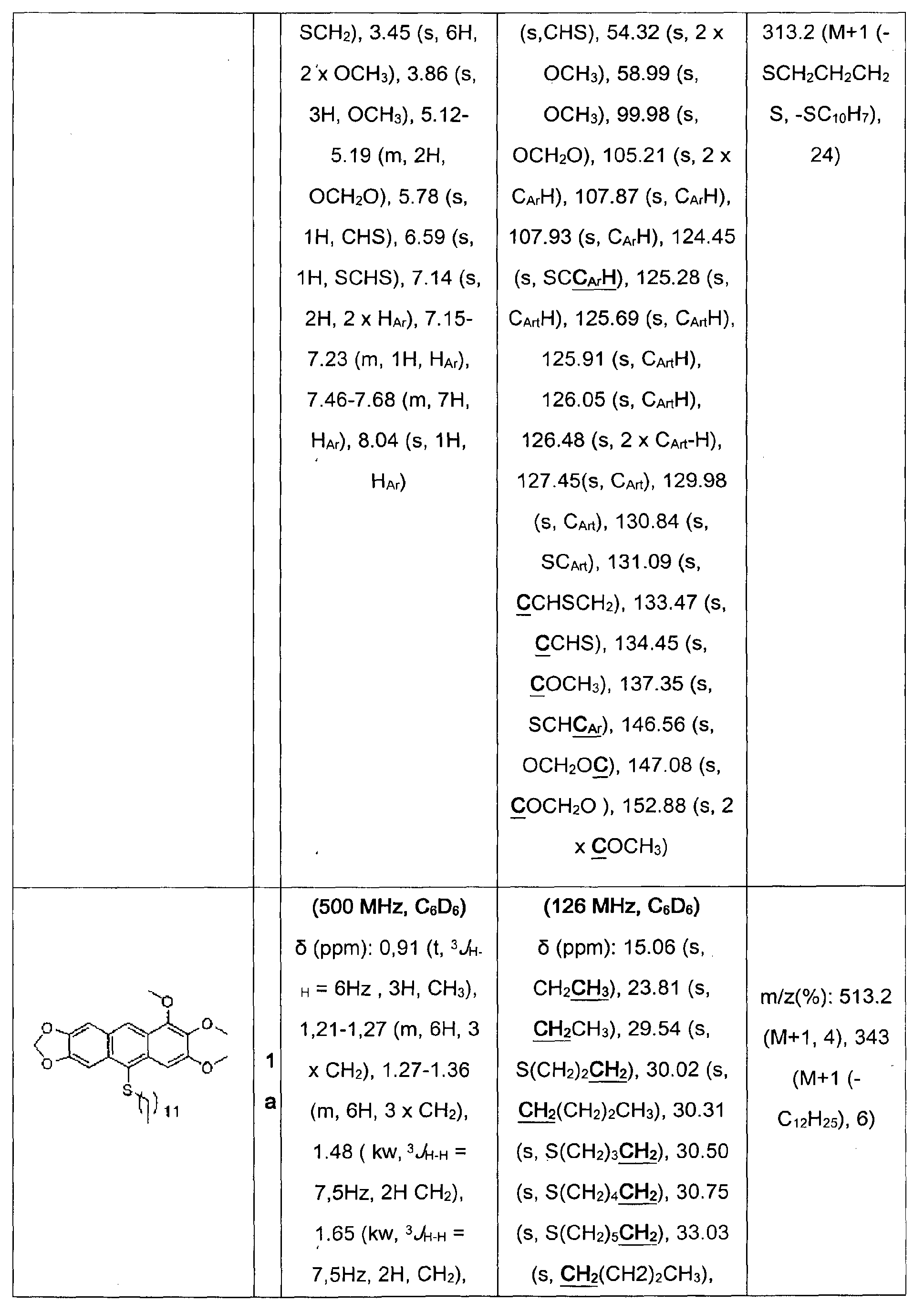

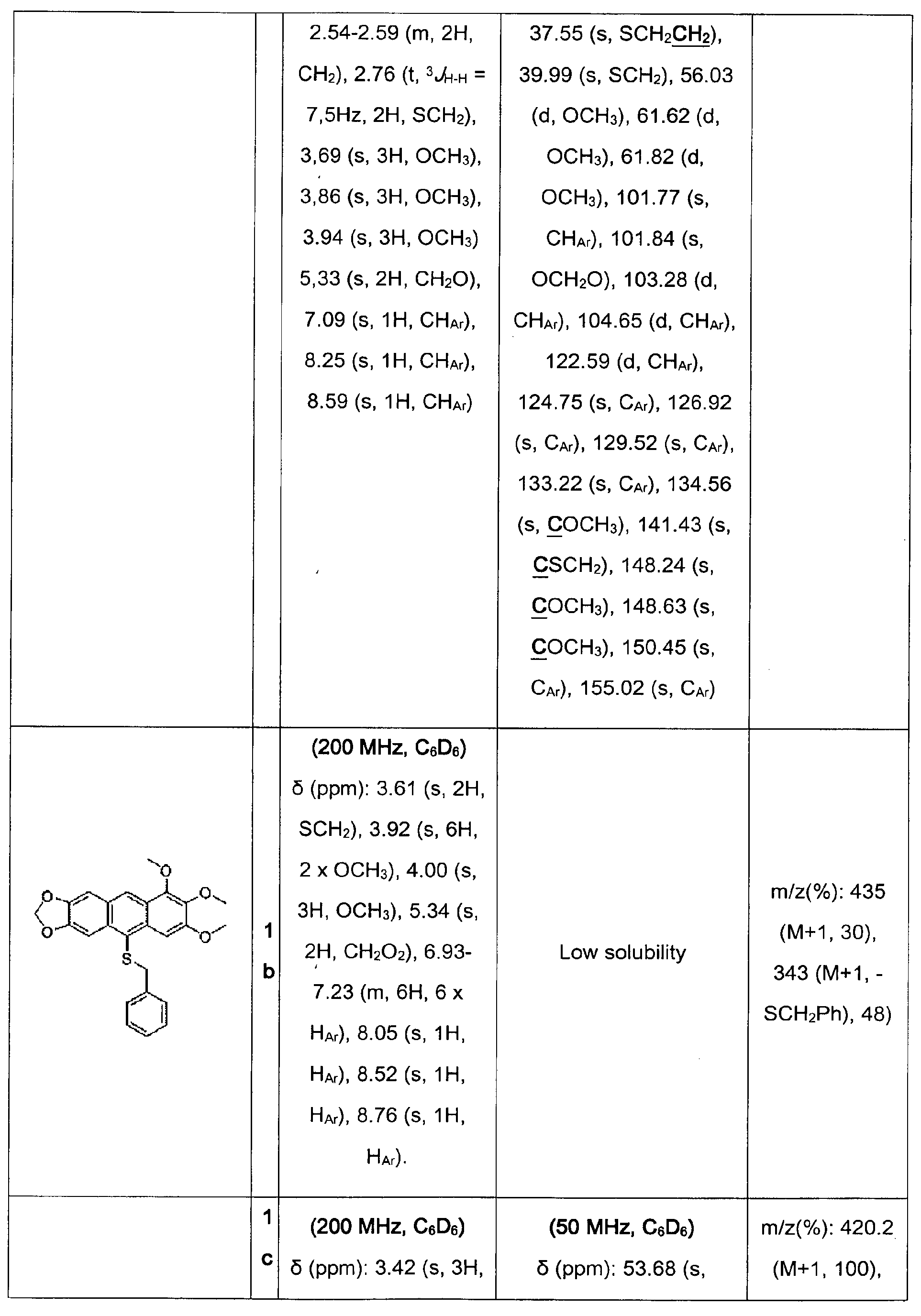

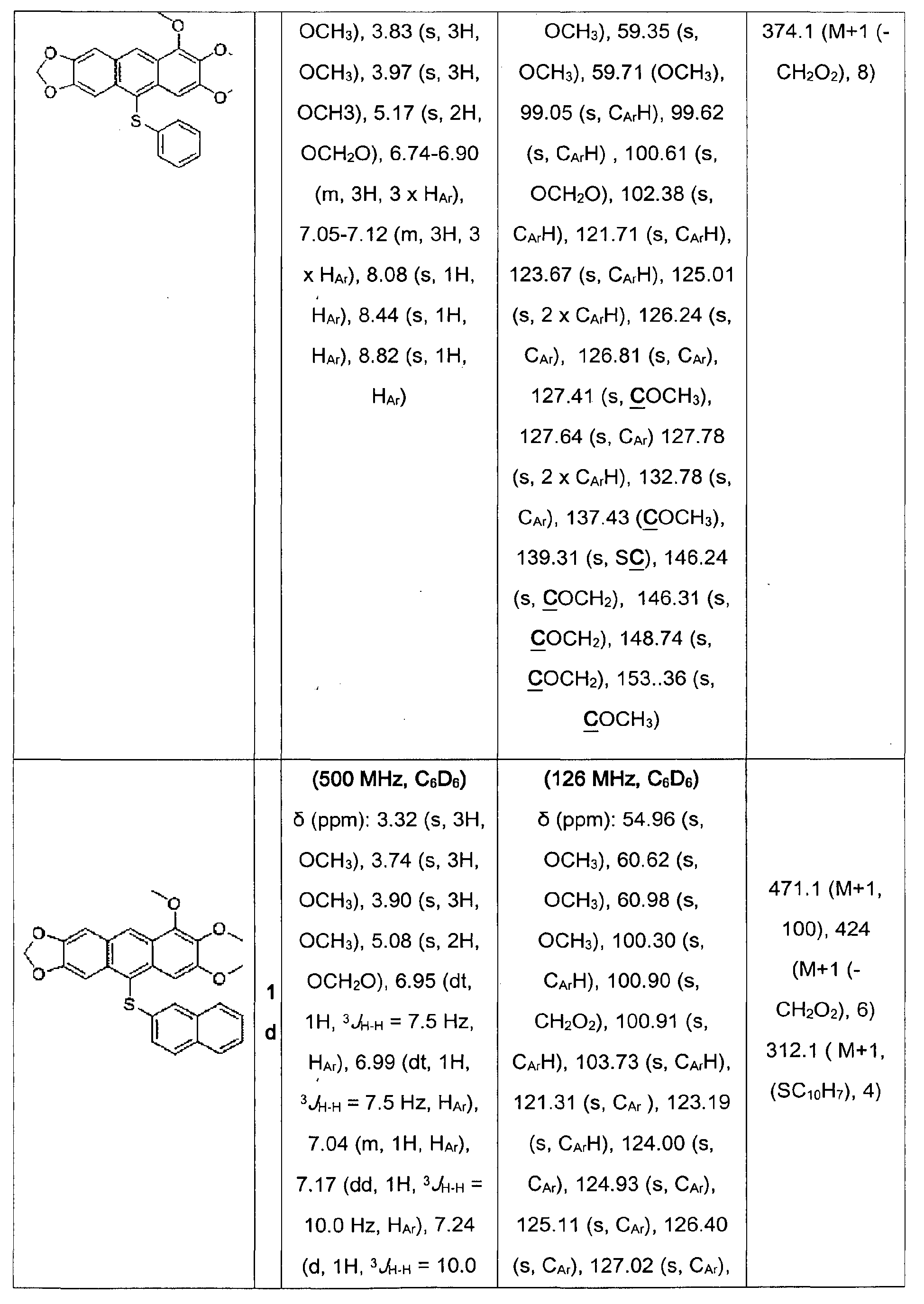

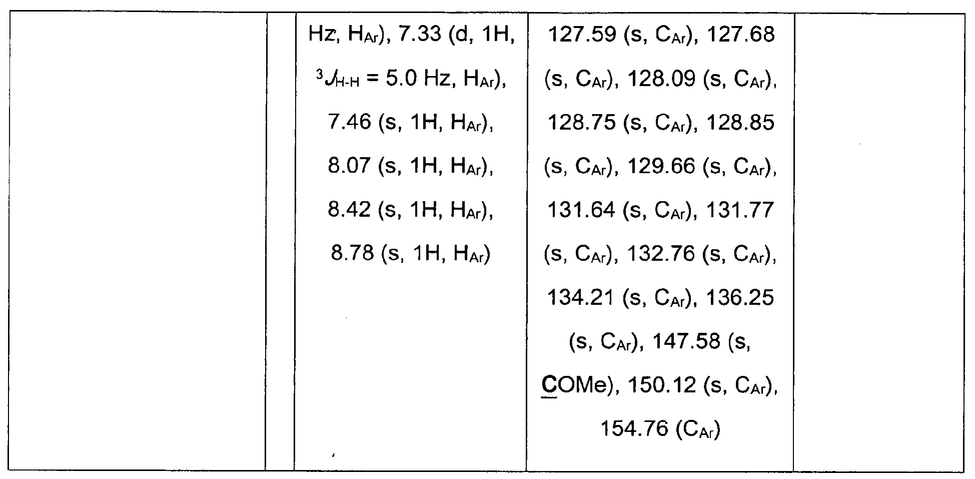

Absorption and fluorescence spectra in chloroform solution.

**Table 2.**

| **Lp** | **F** | **Absorption spectrum** | **Fluorescence sepctrum** |
|---|---|---|---|
| **1** | **1 a** | 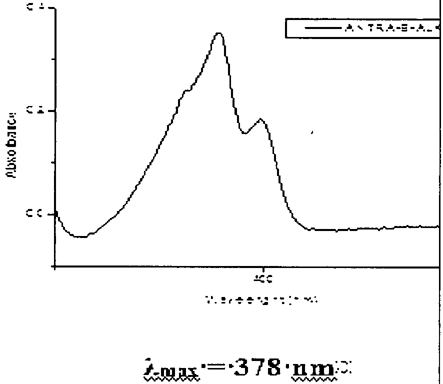 | 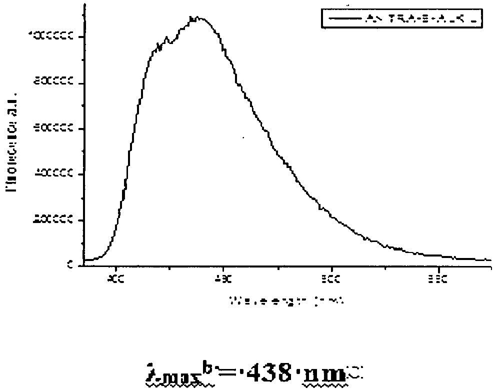 |
| **2** | **1 b** | 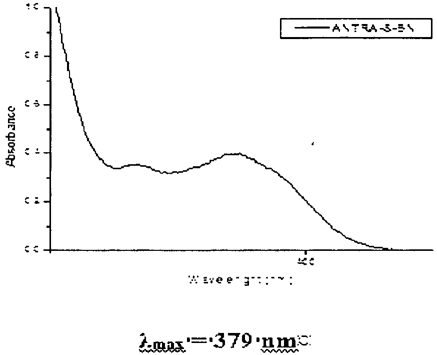 | 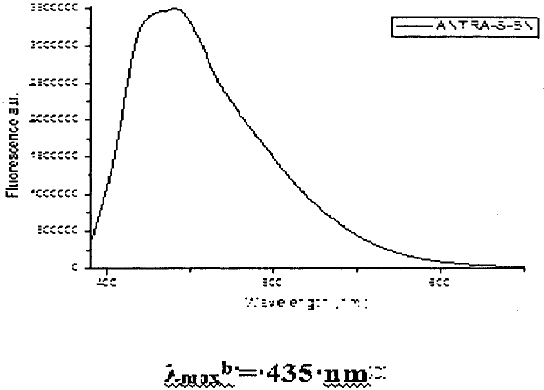 |
| **3** | **1 c** | 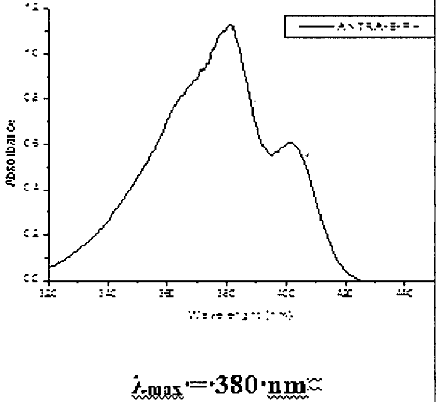 | 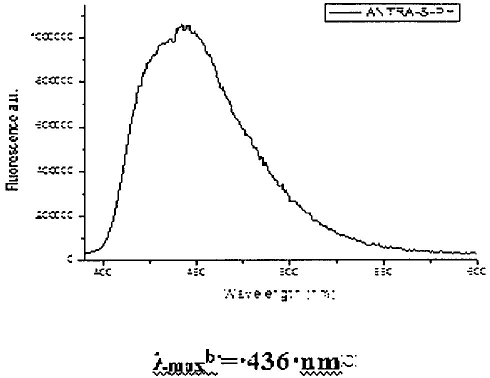 |
| **4 .** | **1 d** | 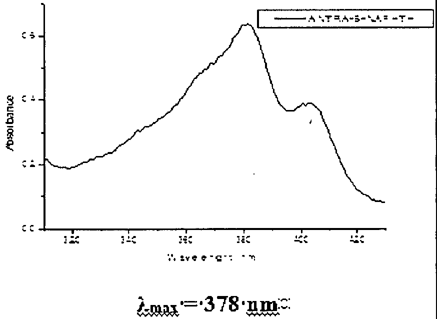 | 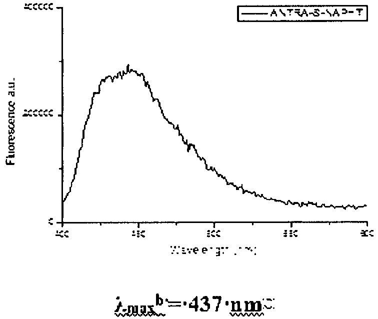 |

| | | | |
|---|---|---|---|
| ^{b} excited at λ = 380nm | | | |

Absorption and fluorescence spectra in benzene solution
**Table 3.**

**Table 3**

| **Lp** | **F** | **Absorption spectrum** | **Fluorescence sepctrum** |
|---|---|---|---|
| **1.** | **1 a** | 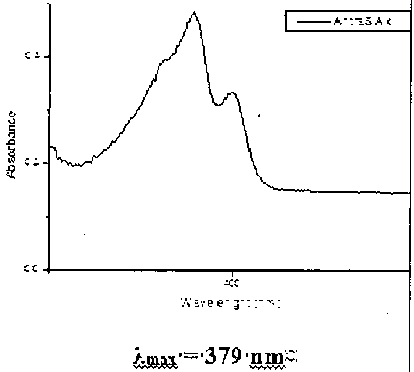 | 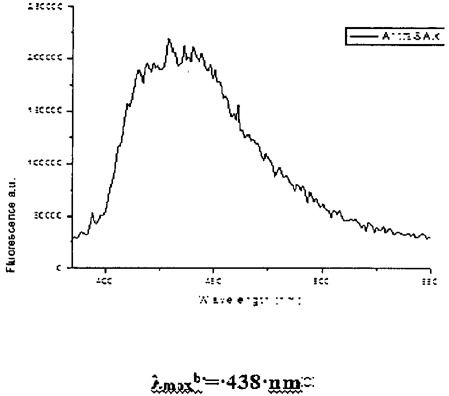 |
| **2.** | **1 c** | 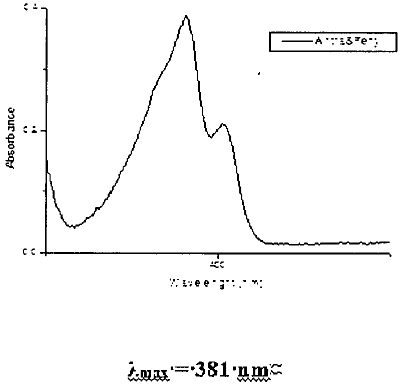 | 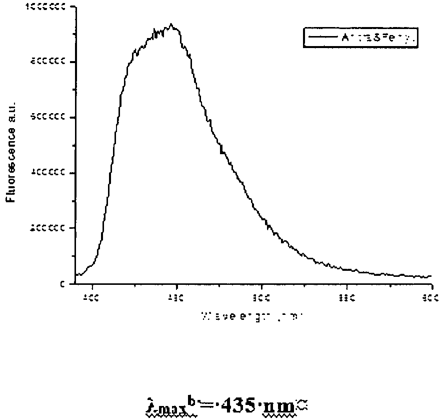 |
| **3.** | **1 d** | | |
| | | 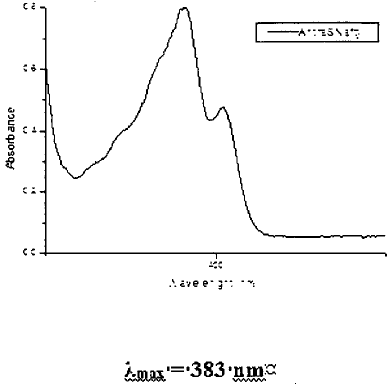 | 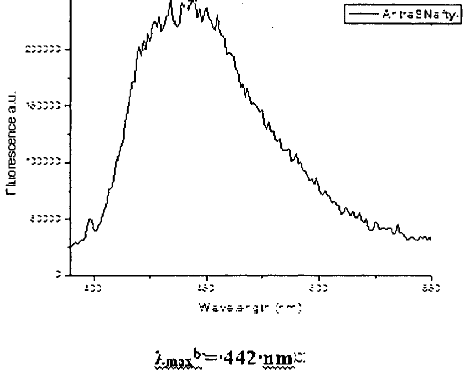 |

| | | | |
|---|---|---|---|
| ^{b} excited at λ = 380nm | | | |

Quantum yields, a comparison of the 10-SR-substituted anthracenes with 10-OR- substituted anthracenes; measurements were carried out in benzene and chloroform solutions.

**Table 4**

| **L p** | **Compound** | **Quantum yields ϕ (CHCl₃)^{a}** | **Quantum yields ϕ (C₆D₆)^{a}** | **L p.** | **Compound** | **Quantum yields ϕ (C₆H₆)^{a}** |
|---|---|---|---|---|---|---|
| **1.** | 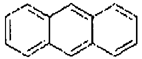 | - | 0.29 | **6.** | 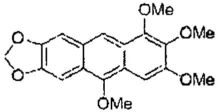 | 0.32 |
| **2.** | 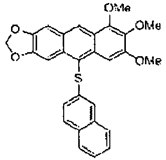 | 0.06 | 0.33 | **7.** | 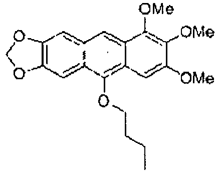 | 0.29 |
| **3.** | 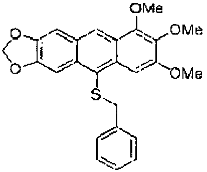 | 0.05 | 0.12 | **8.** | 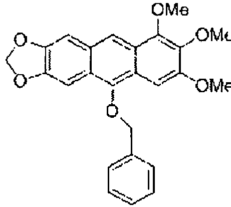 | 0.26 |
| **4.** | 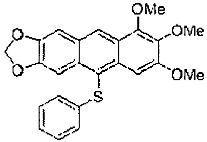 | 0.30 | 0.48 | **9.** | 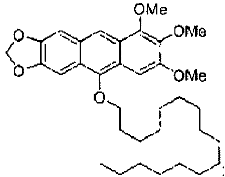 | 0.27 |
| | | 0.17 | 0.17 | | | |

Fluorescence quantum yields, ϕ_{F}, of the RS-substituted anthracenes, calculated using anthracene in the relevant solvent as a reference.

Absorption and fluorescence spectra in solid (thin film obtained by the drop casting method
**Table 5.**

**Table 5**

| **Lp** | **F** | **Absorption spectrum** | **Fluorescence spectrum (emission spectrum, excitation spectrum)** |
|---|---|---|---|
| **1.** | **1a** | 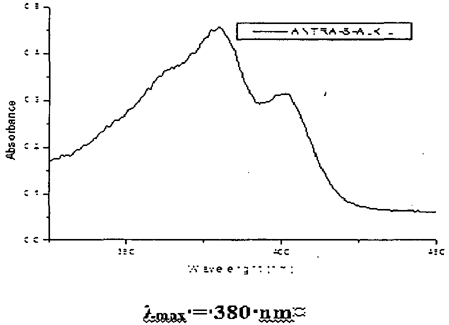 | 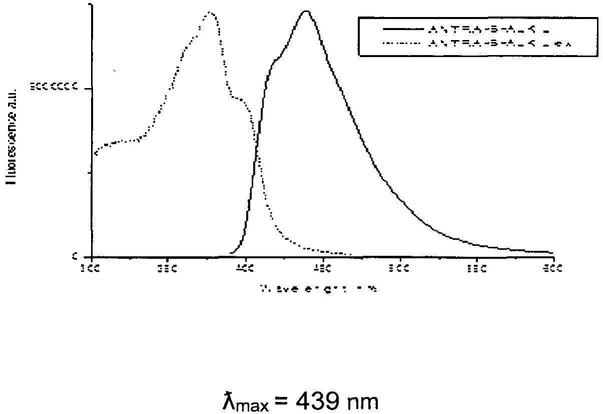 |
| **2.** | **1b** | 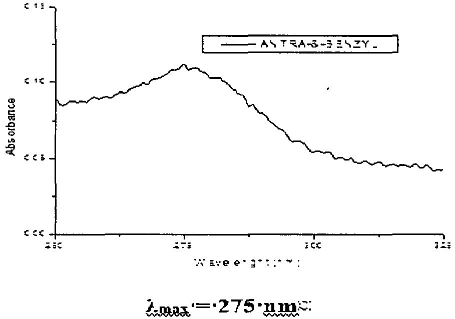 | 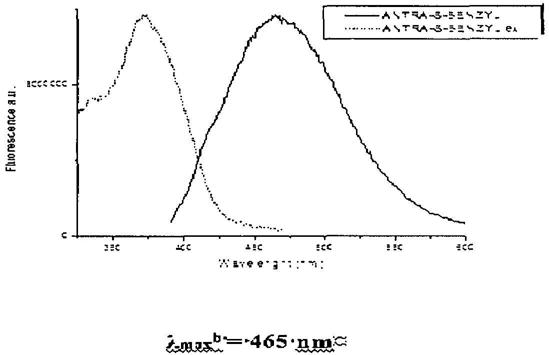 |
| **3 .** | **1c** | 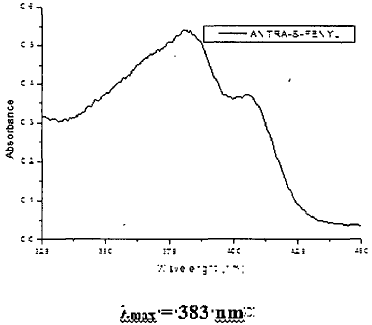 | 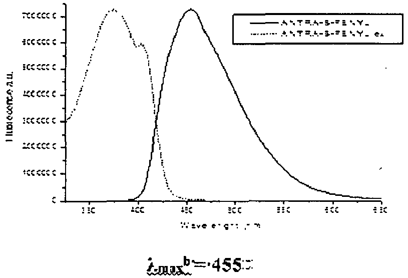 |

-the Stokes shift, calculations based on absorption and fluorescence bands in chloroform, benzene solutions and in solid
**Table 6.**

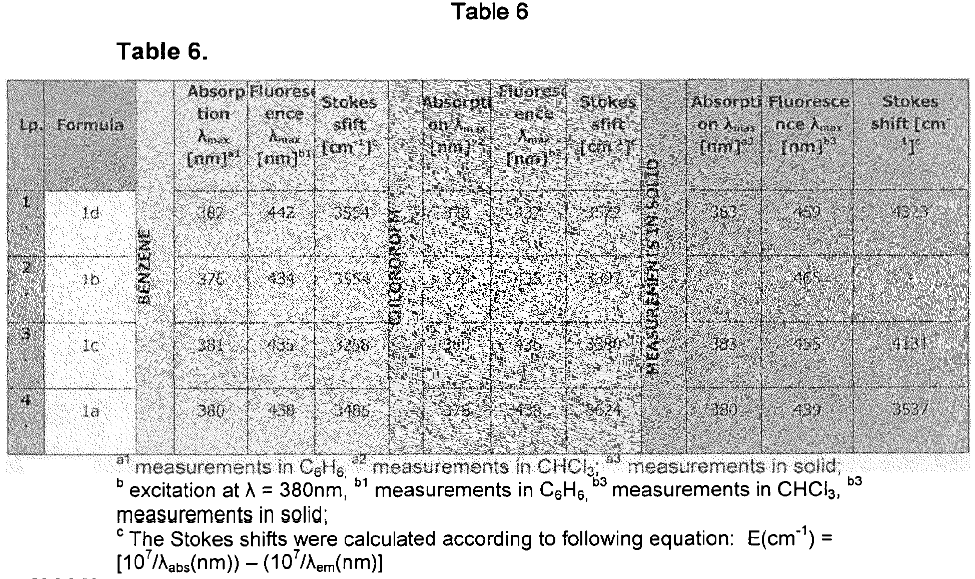

### Citation list

1. Kobayashi K., Masu H., Shuto A., Yamaguchi K., Chem. Mater., 6666, 17, 2005**.**
2. Glass Richard S., Proceedings-Electrochemical Society, 2000-15, 5-8, 2000**.**
3. Tamano Michiko, Nippon Kagaku Kaishi, 4, 731-4, 1985**;**
4. Sipila Kaija, Phosphorus, Sulfur and Silicon and the Related Elements, 177(3), 709-727, 2002**;**
5. Davis Franklin A., Journal of American Chemical Society, 109(11), 3370-7, 1987**.**
6. Majumdar, K. C., Indian Journal of Chemistry, Section B: Organic Chemistry Including Medicinal Chemistry, 25B(12), 1261-2, 1986**.**
7. Charoonniyomporn, Porntip; Tetrahedron Letters, 45(3), 457-459, 2004**.**
8. Pirkle William H., Finn John M., Journal of Organic Chemistry, 48(16), 2779-80, 1983**.**
9. Nerungsi Chakkrapan, Tetrahedron Letters, 51(49) 6392-6395, 2010**.**
10. Saeva F. D., Breslin D. T., From Journal of Organic Chemistry, 54(3), 712-14; 1989**;**
11. Schmidt Luciana C., European Journal of Organic Chemistry, 9, 2210-2214; 2006**;**
12. Node Manabu, Tetrahedron Letters, 23(6), 689-92; 1982**;**
13. Baumgarner Charles D., Helvetica Chimica Acta, 75(2), 480-6; 1992**;**
14. Del Rosso Pablo G., Tetrahedron Letters, 51(51), 6730-6733; 2010;
15. Lee Phil Ho, Journal of Organic Chemistry, 76(3), 760-765; 2011;
16. Tilika V., Latvijas PSR Zinatnu Akademijas Vestis, Kimijas Serija, 6, 738-42; 1990**;**
17. Chan Shiuh-Chuan, Tetrahedron, 65(10), 1977-1981; 2009**.**
18. Takikawa Y., Abe T., Sato R., Takizawa S., Chem. Lett., 25, 1980**;**
19. Harsanyi Michael C., Australian Journal of Chemistry, 48(12), 1949-67, 1995**.**
20. McGrath, Alaina J., From Journal of the American Chemical Society, 132(47), 16759-16761; 2010**.**
21. Majumdar K. C., Indian Journal of Chemistry, Section B: Organic Chemistry Including Medicinal Chemistry, 25B(12), 1261-2; 1986**;**
22. Tamano Michiko, Nippon Kagaku Kaishi, 4, 684-7; 1987**;**
23. Tamano Michiko, Koketsu Jugo, Nippon Kagaku Kaishi, 6, 796-800; 1986**;**
24. Tamano Michiko, Nippon Kagaku Kaishi, 7, 1158-63; 1984**.**
25. Lakshmikantham M. V., Journal of Organic Chemistry, 51 (3), 411-12; 1986**.**
26. Noda T., Shirota Y., J. Am. Chem. Soc., 120, 9714-9715, 1998**;**
27. Sakamoto Y., Komatsu S., Suzuki T., J. Am. Chem. Soc., 123, 4643-4644, 2001**.**
28. Oligotiofeny: Horowitz G., Bachem B., Yassar A., Lang P., Demanze F., Fave J.-L., Garnier F., Chem. Mater., 7, 1337-1341, 1995**;**
29. Sirringhaus H., Brown P. J., Friend R. H., Nielsen M. M., Bechgaard K., Langeveld-Voss B. M. W., Spiering A. J. H., Janssen R. A. J., Meijer E. W., Herwig P., de Leeuw D. M., Nature, 401, 685-688, 1999**;**
30. Facchetti A., Deng Y., Wang A., Koide Y., Sirringhaus H., Marks T. J., Friend R. H., Angew. Chem., Int. Ed., 39, 4547-4551, 2000**;**
31. Izumi T., Kobashi S., Takimiya K., Aso Y., Otsubo T., J. Am. Chem. Soc., 125, 5286-5287, 2003;
32. Janzen D. E., Burand M. W., Ewbank P. C., Pappenfus T. M., Higuchi H., da Silva Filho D. A., Young V. G., Bre'das J.-L., Mann K. R., J. Am. Chem. Soc., 126, 15295-15308, 2004;
33. Other organosulfur aromatic compounds : Laquindanum J. G., Katz H. E., Lovinger A. J., J. Am. Chem. Soc., 120, 664-672, 1998**;**
34. Mas-Torrent M., Durkut M., Hadley P., Ribas X., Rovira C., J. Am. Chem. Soc., 126, 984-985, 2004;
35. Takimiya K., Kunugi Y., Konda Y., Niihara N., Otsubo T., J. Am. Chem. Soc., 126, 5084-5085, 2004**.**
36. Adam D., Schuhmacher P., Simmerer J., Ha¨ussling L., Siemensmeyer K., Etzbach K. H., Ringsdorf H., Haarer D., Nature, 371, 141-143, 1994**.**
37.¹⁷ Ranken P. F., McKinnie B. G., Synthesis, 117, 1984; Do Q. T., Elothmanli D., Le Guillanton G., Tetrahedron Letters, 39, 4657, 1988**.**

## Claims

1. New 10-thiosubstituted pentahydroxyanthracene derivatives represented by the general formula **1,** 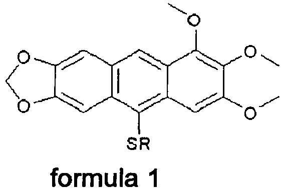 wherein R- is - alkyl having 1 to 12 carbon atoms, benzyl, phenyl or napthyl, especially represented by formulas **1a to 1d.**

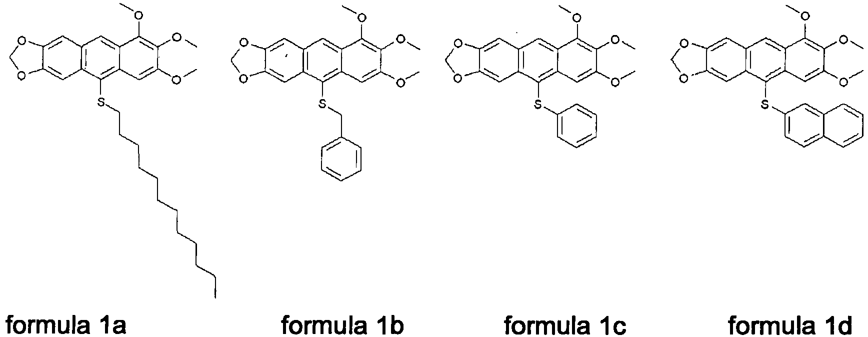

2. The intermediates of the general formula **2,** 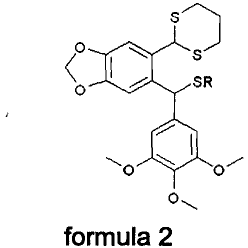 wherein R- is alkyl having 1 to 12 carbon atoms, benzyl, phenyl or napthyl, especially represented by formulas **2a to 2d.**

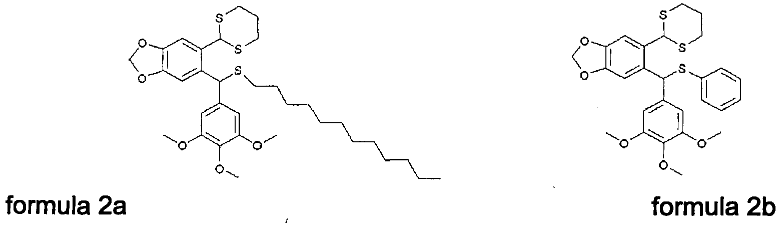

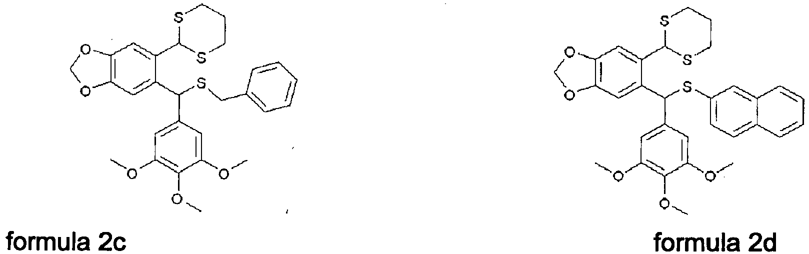

3. A method for the preparation of fused aromatic hydrocarbons of the formula 1, 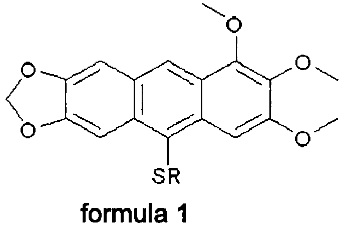 wherein R- is - alkyl having 1 to 12 carbon atoms, benzyl, phenyl or napthyl, **characterized in that** is carried out the reaction of transformation of diarylmethanethiols derivatives of the formula **2** under mild conditions, in the presence of FeCl₃/KI, in a boiling organic solvent, preferably methanol, in a single reaction vessel, without isolation of the intermediates, to give a compound of the formula **1** in good yields (Scheme I).

4. A method according to the claim 3, **characterized in that**, a mixture of FeCl₃/KI in a 1:1 ratio is used, which allows to perform the above chemical transformation in a short time, efficiently and without the need for purification of the final products.

## Patentansprüche

1. Neue 10-Thiosubstituierte Pentahydroxanthracen-Deritative, die mit der allgemeinen Formel 1 dargestellt werden, 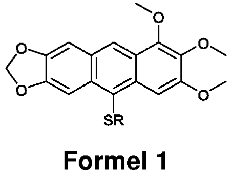 wo R- bedeutet - Alkyl, das 1÷12 Atome vom Kohlenstoff, Benzyl, Phenyl, oder Naphtyl enthält , die in Formeln von 1 a bis 1 d dargestellt werden.

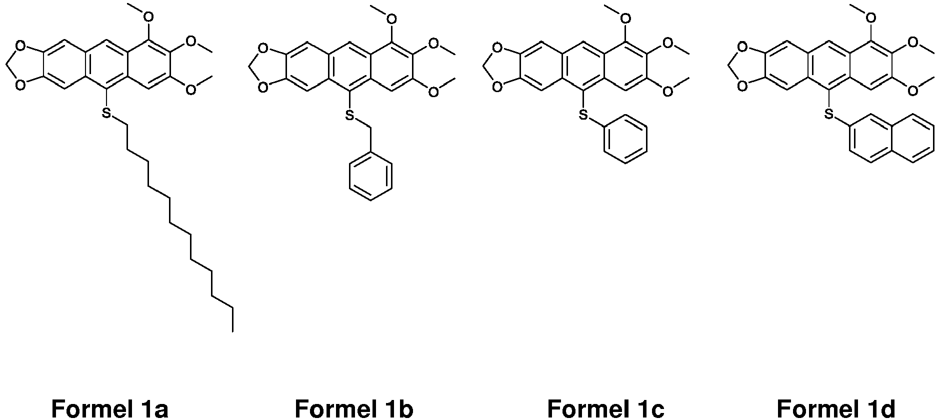

2. Indirekte Verbindungen mit der allgemeinen Formel **2,** 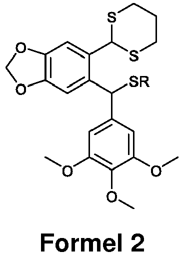 wo R- bedeutet - Alkyl, das 1÷12 Atome vom Kohlenstoff, Benzyl, Phenyl, oder Naphtyl enthält , die in Formeln von 2a bis 2d dargestellt werden.

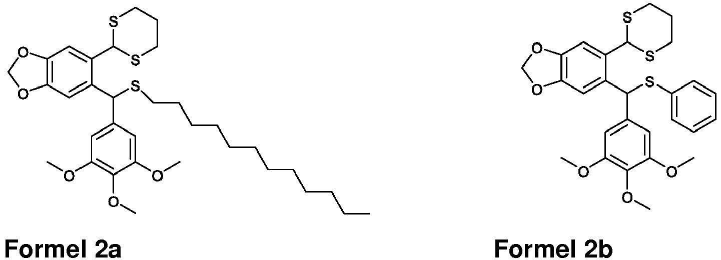

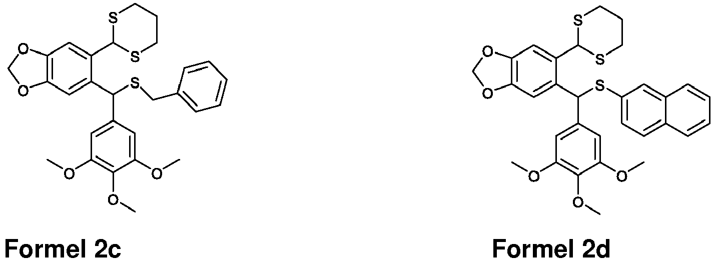

3. Verfahren zur Erzeugung der kondensierten aromatischen Kohlenwasserstoffe mit der Formel 1, 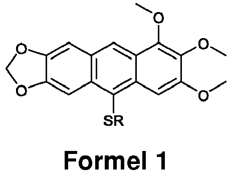 wo R- bedeutet - Alkyl, das 1÷12 Atome vom Kohlenstoff, Benzyl, Phenyl, oder Naphtyl enthält, **dadurch gekennzeichnet, dass** es die Reaktion der Transformation von Diarylmethanthiol-Deritative durchgeführt wird, und zwar mit der Formel **2** bei zarten Verhältnissen, in Anwesenheit von FeCl₃/KI, im kochenden organischen Lösungsmittel, vorteilhaft für Methanol, in einem Reaktionsgefäß, ohne indirekte Produkte abzuteilen, bis die Verbindung mit der Formel **1** mit guten Effizienten erhalten wird **(Schema I).**

4. Verfahren gemäß des 3. Patentananspruchs, **dadurch gekennzeichnet, dass** die Mischung FeCl₃/KI im Verhältnis 1:1 eingesetzt wird, was die Durchführung der oben genannten chemischen Transformation in der kurzen Zeit, effizient und ohne Notwendigkeit ermöglicht, die Endprodukte zu reinigen.

## Revendications

1. Nouveaux dérivés 10-Tiosubstitués de penta hydroxy anthracène indiqués par la formule générale 1, 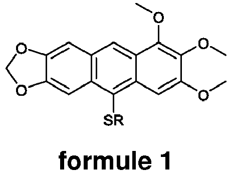 où **R-** désigne - un alkyl contenant 1÷12 atomes de carbone, un benzyle, un phényle ou un naphtyle, en particulier montrés par les formules de 1 a à 1 d.

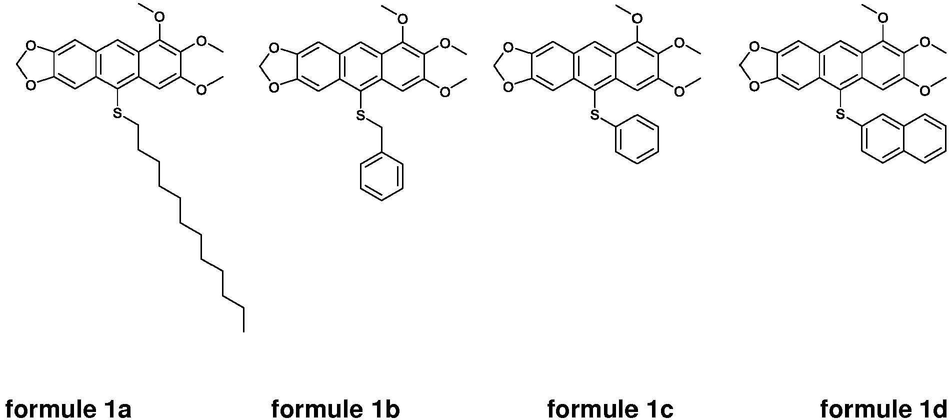

2. Produits indirects à la formule générale **2,** 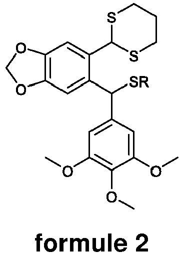 où **R-** désigne - un alkyl contenant 1÷12 atomes de carbone, un benzyle, un phényle ou un naphtyle, en particulier montrés par les formules de 2a à 2d.

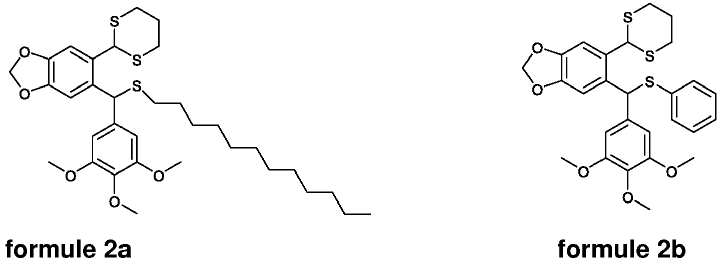

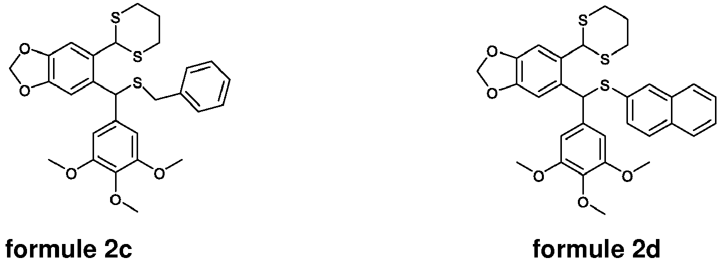

3. Processus d'obtention de hydrocarbures aromatiques condensés à la formule **1,** 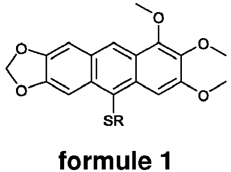 où **R-** désigne - un alkyl contenant 1÷12 atomes de carbone, un benzyle, un phényle ou un naphtyle, caractéristique parce que la réaction de transformation de dérivés de diarylomethanothioles, à la formule **2** dans les conditions douces, en présence de FeCl₃/KI, en solvant organique bouillant, de préférence le méthanol, dans un seul récipient de réaction, sans isoler des produits intermédiaires, jusqu'à l'obtention de produit à la formule **1** avec de bons rendements **(Modèle I).**

4. La façon selon restr. 3, caractéristique parce qu'on utilise le mélange de FeCl₃/KI dans le ratio 1:1, ce qui rend possible la transformation ci-dessus dans un court délai, de façon efficace, et sans besoin de purifier des produits finales.
